# FASCICULE DE BREVET EUROPEEN

(11) **EP 1 448 676 B1**
(45) Date de publication et mention de la délivrance du brevet: **23.05.2012**
(21) Numéro de dépôt: 02790550.4
(22) Date de dépôt: 08.11.2002
(51) Int. Cl.: C08H 1/00, C08H 1/06

(54) **PROCEDE DE PREPARATION D'UN PRINCIPE ACTIF A BASE DE PROTEINES POLYMERISEES, PRINCIPE ACTIF ET COMPOSITION COSMETIQUE**
VERFAHREN ZUR HERSTELLUNG VON EINEM WIRKSTOFF AUF DER BASIS VON POLYMERISIERTEN PROTEINEN, WIRKSTOFF UND KOSMETISCHE ZUSAMMENSETZUNG
METHOD FOR PREPARING AN ACTIVE PRINCIPLE BASED ON POLYMERIZED PROTEINS, ACTIVE PRINCIPLE AND COSMETIC COMPOSITION

(30) Priorité: 09.11.2001 FR 0114545
(43) Date de publication de la demande: 25.08.2004
(73) Titulaire: Société Industrielle Limousine d'Application Biologique (SILAB), 19130 Objat (FR)
(72) Inventeur: PAUFIQUE, Jean-Jacques, F-19130 Objat (FR)
(74) Mandataire: Fantin, Laurent
(86) Numéro de dépôt international: PCT/FR2002/003833
(87) Numéro de publication internationale: WO 2003/040216

(56) Documents cités:
- DATABASE WPI Week 198526 Derwent Publications Ltd., London, GB; AN 1985-156875 XP002206976 "Cosmetic prepn. for improving skin roughness - contg. soluble collagen deriv. and brown colour extracted from brown sugar" & JP 60 089410 A (SHISEIDO CO LTD), 2 mai 1985 (1985-05-02)
- PATENT ABSTRACTS OF JAPAN vol. 1995, no. 09, 31 octobre 1995 (1995-10-31) & JP 07 165526 A (MIKIMOTO PHARMACEUT CO LTD), 27 juin 1995 (1995-06-27) & DATABASE CHEMABS [en ligne] CHEMICAL ABSTRACTS SERVICE, COLUMBUS, OHIO, US; "Conchiolin hydrolyzate as a material for skin-moisturizing cosmetics and its preparation"

## Description

La présente invention a trait à un procédé de préparation d'un principe actif à base de protéines polymérisées de haut poids moléculaire d'intérêt, notamment en cosmétique, au principe actif susceptible d'être obtenu par un tel procédé, à une composition, en particulier cosmétique, contenant ledit principe actif et à une méthode de traitement cosmétique consistant à appliquer sur la peau une telle composition.

Par leur aptitude à s'adsorber et à s'étaler à la surface de la peau, les protéines y forment un film élastique, cohésif, lisse et continu. Les propriétés filmogènes, tenseurs, lissantes et plastifiantes des protéines sont ainsi particulièrement recherchées.

Mais les protéines natives, notamment les protéines de haut poids moléculaire, posent des problèmes de formulation et en particulier de solubilité en milieux aqueux et de stabilité. Elles sont notamment peu solubles et stables en émulsion. Sur la peau ces protéines forment des agrégats disposés de manière anarchique ne formant pas un film protéique continu et générateur de propriétés lissantes.

La demande JP 60089410 décrit l'utilisation d'hydrolysat de collagène pour lutter contre la rugosité de la peau.

Le demandeur a donc identifié le besoin d'un procédé de préparation de protéines de haut poids moléculaire qui soient aptes à être formulées, notamment dans des compositions cosmétiques, et qui conservent leurs propriétés bénéfiques pour la peau.

La demande WO 0228360 décrit un procédé d'obtention de protéines polymérisées, mais les protéines obtenues n'ont pas une activité tenseur satisfaisante et adaptée à ce besoin.

Aussi la présente invention concerne un procédé de préparation d'un principe actif de protéines polymérisées de haut poids moléculaire, ledit procédé comprenant les étapes consécutives suivantes consistant à
(i) solubiliser en phase aqueuse une matière riche en protéines ;
(ii) hydrolyser par voie enzymatique avec une protéase de type endoprotéase, exoprotéase, exopeptidase, protéase-peptidase, aminopeptidase ou carbopeptidase ou un mélange de ces enzymes de manière contrôlée et limitée la solution obtenue en (i) avec un taux d'hydrolyse enzymatique choisi de manière à maintenir des protéines monomères de masse molaire de 20000 Da à 300000 Da ; optionellement, inactiver le(s) enzyme(s) utilisée(s);
(iii) séparer les phases solubles et insolubles ; et
(iv) polymériser les protéines par ajout d'un agent polymérisant multifonctionnel choisi parmi les polychlorures d'acides, les polyanhydrides d'acides, les polyisocyanates et les polythioisocyanates.

Le procédé de la présente invention permet la préparation de protéines de haut poids moléculaire polymérisées d'intérêt, notamment cosmétique. Les protéines polymérisées, ainsi obtenues garantissent notamment une meilleure solubilité et stabilité.

Un des avantages du procédé de la présente invention est de permettre, à partir d'une matière riche en protéines, la création d'un réseau tridimensionnel de masse molaire élevée capable d'induire une architecture moléculaire parfaitement adaptée à l'amélioration des propriétés biomécaniques de la peau.

D'autres caractéristiques complémentaires du procédé de la présente invention sont les suivantes :
- la matière riche en protéines est de préférence choisie parmi des farines, concentrats, isolats, tourteaux de protéines naturelles d'origine végétale et/ou animale, et/ou marine ;
- solubilisation en phase aqueuse de ladite matière à raison de 50 g/l à 500 g/l ;
- l'hydrolyse est de préférence réalisée à pH acide, de préférence de pH 4 à pH 7, à une température d'environ 40°C à environ 80°C ;
- comme exemples d'enzymes on peut citer une protéase de type endoprotéase, exoprotéase, exopeptidase, protéase-peptidase, aminopeptidase ou carbopeptidase, ou un mélange de ces enzymes ; le(s) enzyme(s) utilisée(s) sont alors inactivée(s) par exemple par augmentation de la température du milieu à une température comprise entre 50°C et 80°C;
- l'agent de polymérisation est de préférence un agent polymérisant multifonctionnel choisi dans le groupe des initiateurs de polymérisation comprenant les polychlorures d'acides, les polyanhydrides d'acides, les polyisocyanates, les polythioisocyanates ; l'étape de polymérisation est réalisée, de préférence, avec un taux d'agent polymérisant de 10 g/l à 100 g/l, choisi de manière à obtenir un taux de protéines polymérisées supérieur à environ 30% ;
- la séparation des protéines polymérisées est réalisée de préférence par filtration, décantation, centrifugation et concentration.

La présente invention a encore pour objet le principe actif susceptible d'être obtenu par le procédé tel qu'il vient d'être décrit.

Ledit principe actif est caractérisé en ce qu'il comprend
- un taux de matières sèches, tel qu'obtenu par passage à l'étuve à 105°C jusqu'à obtention d'un poids constant, d'environ 50 g/l à environ 500 g/l, de préférence d'environ 50 g/l à environ 250 g/l ;
- un pH, tel qu'obtenu par la méthode potentiométrique, à température ambiante, d'environ 2 à environ 10, de préférence d'environ 4 à environ 10, plus particulièrement d'environ 7 à environ 8 ;
- un taux de protéines, tel que mesuré par la méthode de Biuret à 540 nm, d'environ 40 g/l à environ 400 g/l ; de préférence ce taux est d'environ 40 g/l à 200 g/l ;
- la taille des protéines est définie par un profil chromatographique ; la masse molaire des différentes espèces moléculaires est estimée par chromatographie F.P.L.C (Fast Protein Liquid Chromatography) ; la colonne de filtration, de dénomination commerciale «Superdex 200 », par exemple, est étalonnée avec des marqueurs de masse moléculaire connue tels que : cytochrome C, 12500 Da (Daltons), alcool déshydrogénase, 15000 Da, anhydrase carbonique, 29000 Da, albumine bovine, 66000 Da, apoferritine, 443000 Da, thyroglobuline, 669000 Da ; la détection des composants élués est effectuée dans l'ultra-violet à 280 nm ; un pourcentage de protéines déterminé contenues dans chaque fraction, fraction F1 = masse molaire supérieure à 500000 Da, fraction F2 = masse molaire entre 150000 et 500000 Da, et fraction F3 = masse molaire inférieure à 150000 Da ; de préférence ce pourcentage est d'environ 30% à environ 60% dans la fraction F1.

La présente invention a encore pour objet une composition cosmétique comprenant au moins un principe actif préparé selon ledit procédé.

Ladite composition destinée en particulier à procurer un effet immédiat sur les propriétés biomécaniques de la peau peut se présenter sous toute forme galénique appropriée. De préférence, la composition contenant ledit principe actif se présente sous la forme d'une crème, d'un gel, d'un onguent, d'une lotion, d'un lait, d'une émulsion ou d'une solution.

Ledit principe actif est présent dans la composition en % (p/p) d'environ 0,5% à environ 20%.

La présente invention a également pour objet une méthode de traitement cosmétique de la peau consistant à appliquer une composition comprenant ledit principe actif obtenu selon le procédé de l'invention, ce par quoi un effet lissant, tenseur immédiat de la peau est obtenu.

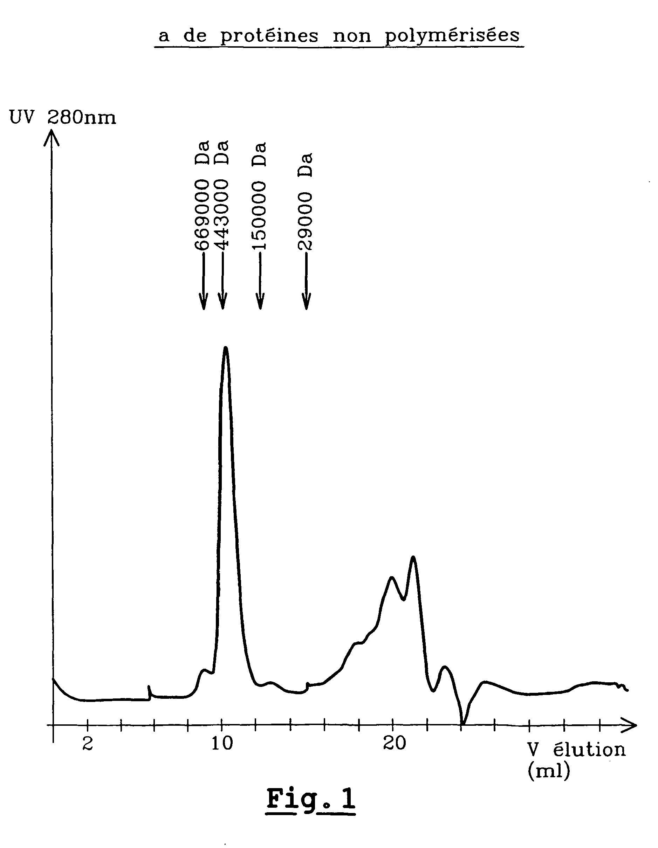

La présente invention va maintenant être décrite de manière plus détaillée, dans ses caractéristiques et avantages à l'aide d'exemples de réalisation donnés à titre purement illustratif et en référence aux dessins annexés dans lesquels
la Figure 1 représente un profil chromatographique de protéines obtenues après une étape d'hydrolyse enzymatique contrôlée et modérée avec un taux d'enzyme fixé à 1 équivalent sans étape de polymérisation (témoin « protéines a ») ;

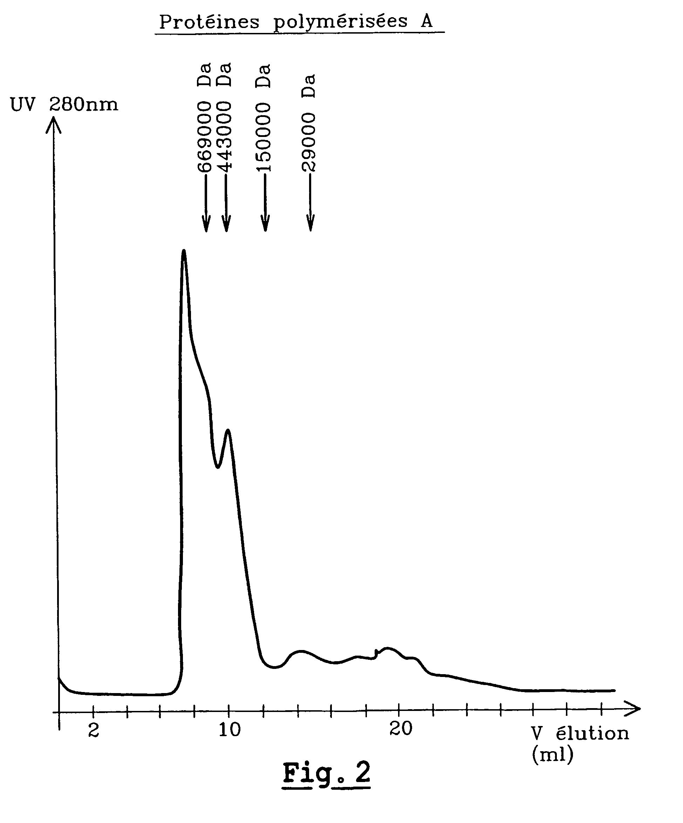

la Figure 2 représente le profil chromatographique des protéines obtenues par le procédé selon l'invention comprenant une étape d'hydrolyse enzymatique contrôlée et modérée avec un taux d'enzyme fixé à 1 équivalent et une étape de polymérisation avec un taux d'agent polymérisant fixé à 1 équivalent (« protéines polymérisées A ») ;

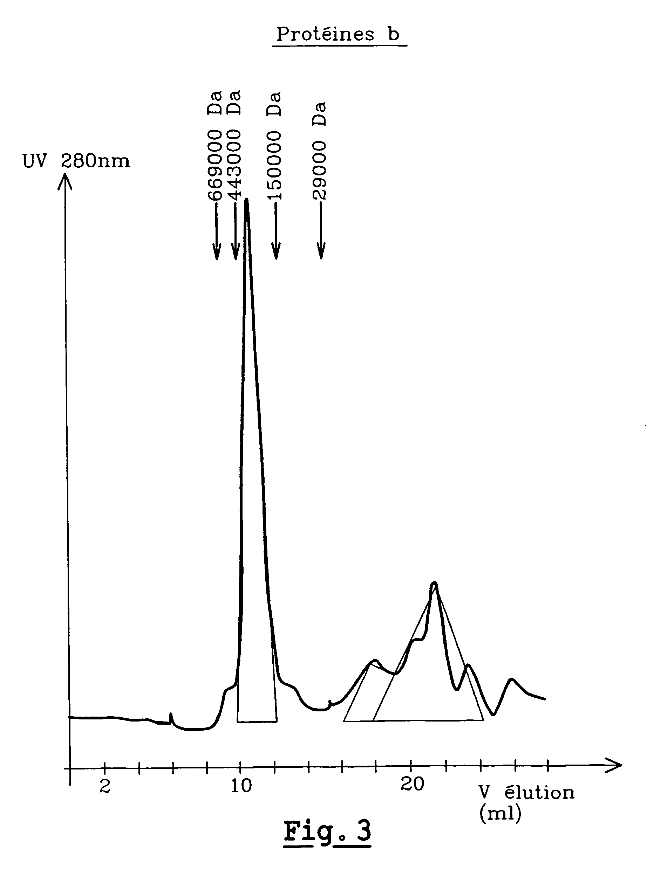

La Figure 3 représente le profil chromatographique des protéines b obtenues par le procédé comprenant une étape d'hydrolyse enzymatique contrôlée et modérée avec un taux d'hydrolyse fixé à 1/12 équivalent (« protéines b »);

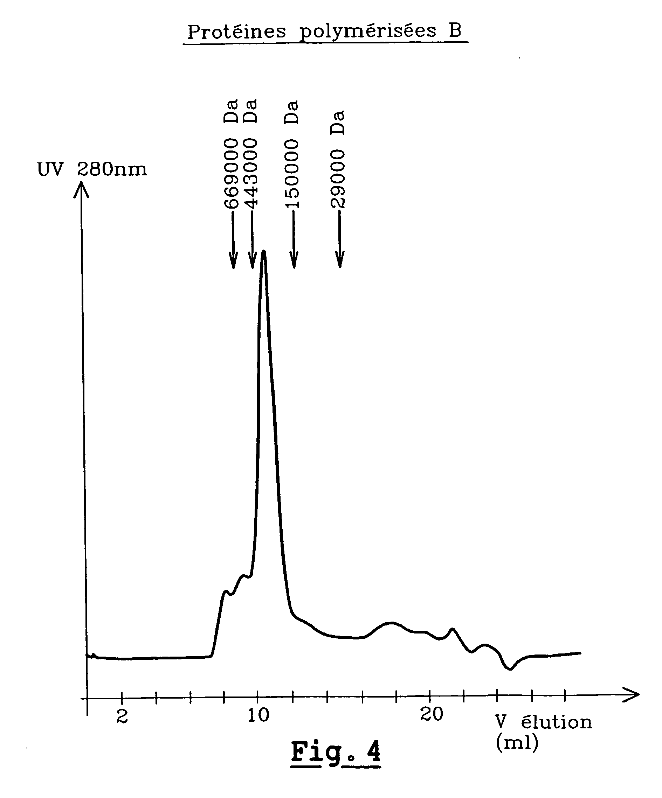

la Figure 4 représente le profil chromatographique des protéines obtenues par le procédé selon l'invention comprenant une étape d'hydrolyse enzymatique contrôlée et modérée avec un taux d'enzyme fixé à 1/12 équivalent et une étape de polymérisation avec un taux d'agent polymérisant fixé à 1 équivalent (« protéines polymérisées B »;

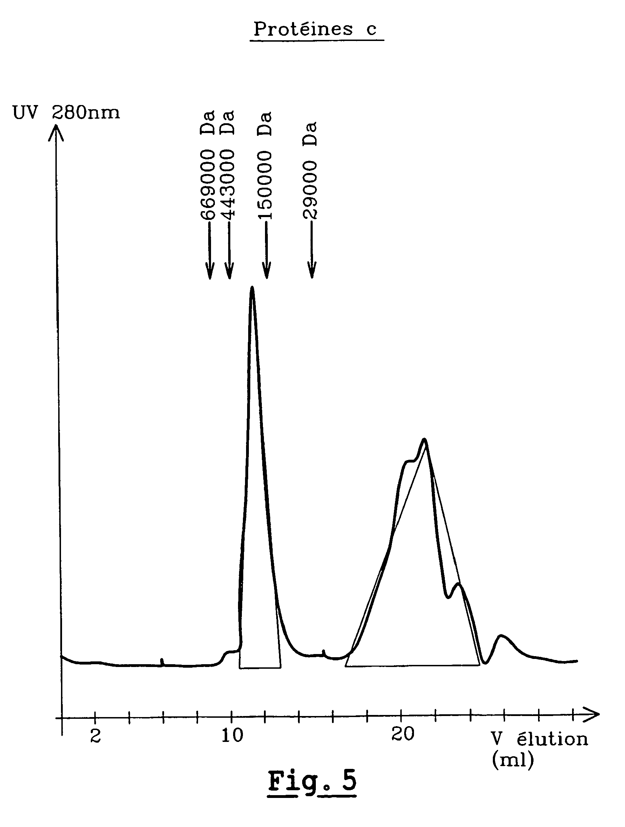

la Figure 5 représente le profil chromatographique des protéines obtenues par le procédé selon l'invention comprenant une étape d'hydrolyse enzymatique contrôlée et modérée avec un taux d'hydrolyse fixé à 10/3 équivalent (« protéines c ») ;

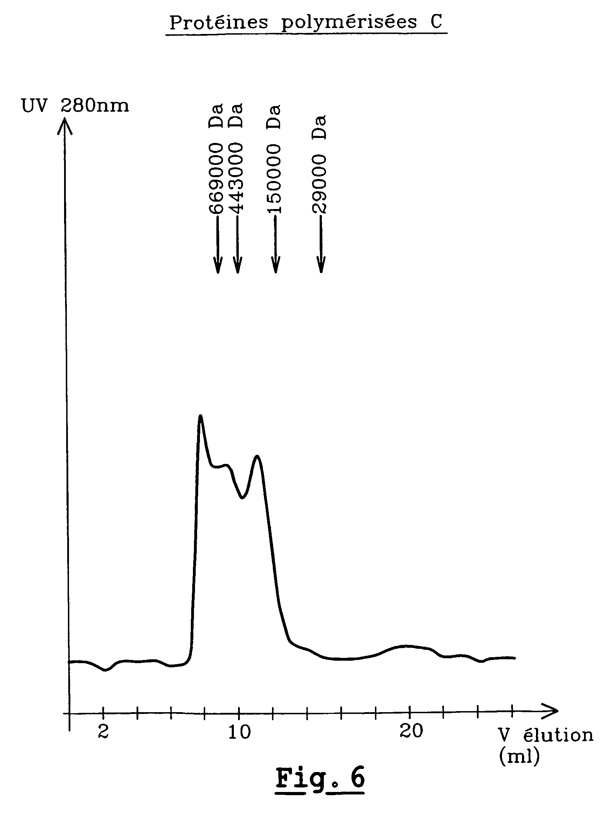

la Figure 6 représente le profil chromatographique des protéines obtenues par le procédé selon l'invention comprenant une étape d'hydrolyse enzymatique contrôlée et modérée avec un taux d'enzyme fixé à 10/3 équivalent et une étape de polymérisation avec un taux d'agent polymérisant fixé à 1 équivalent (« protéines polymérisées C »);

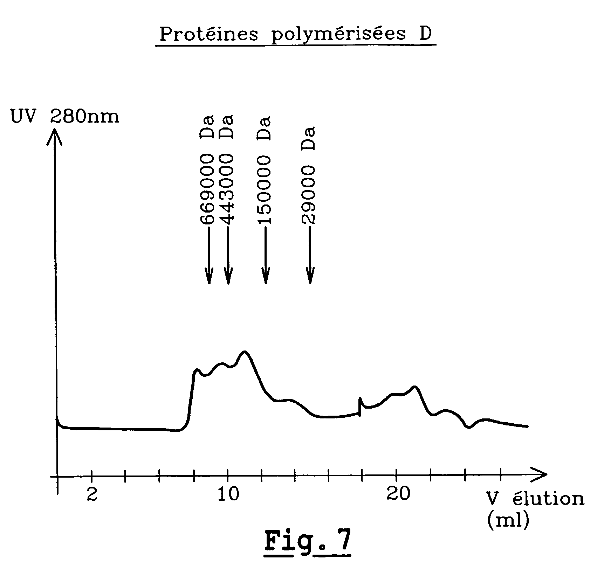

la Figure 7 représente le profil chromatographique des protéines obtenues par le procédé selon l'invention comprenant une étape d'hydrolyse enzymatique contrôlée et modérée avec un taux d'enzyme fixé à 1 équivalent et une étape de polymérisation avec un taux d'agent polymérisant fixé à 5/8 équivalent (« protéines polymérisées D ») ;

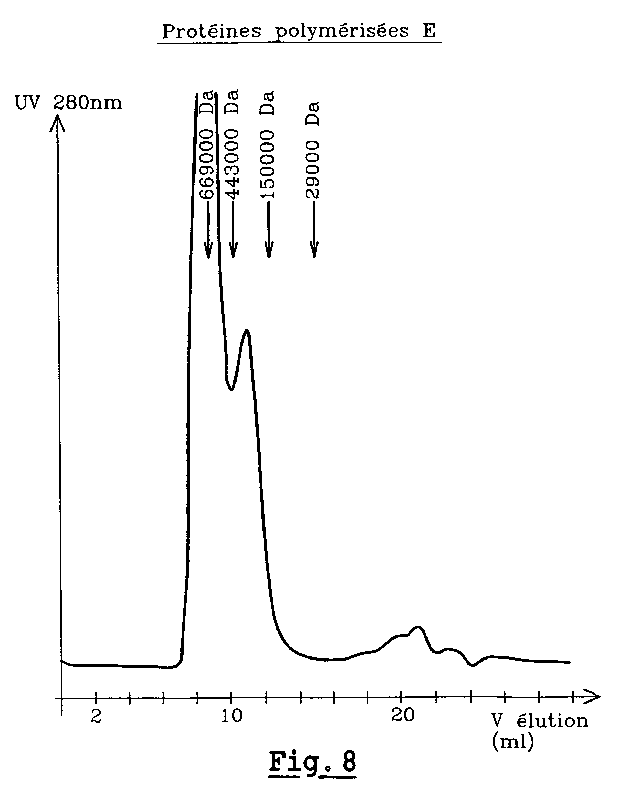

la Figure 8 représente le profil chromatographique des protéines obtenues par le procédé selon l'invention comprenant une étape d'hydrolyse enzymatique contrôlée et modérée avec un taux d'enzyme fixé à 1 équivalent et une étape de polymérisation avec un taux d'agent polymérisant fixé à 5/4 équivalent (« protéines polymérisées E »); et

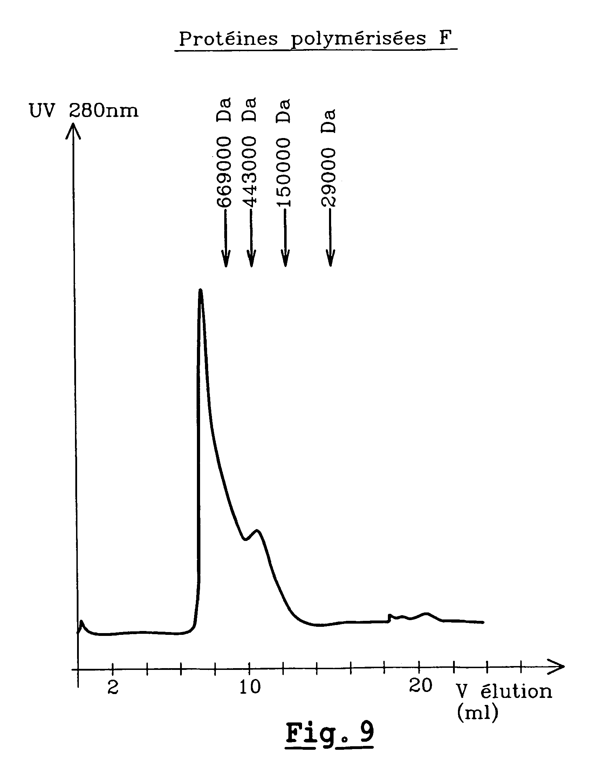

la Figure 9 représente le profil chromatographique des protéines obtenues par le procédé selon l'invention comprenant une étape d'hydrolyse enzymatique contrôlée et modérée avec un taux d'enzyme fixé à 10/3 équivalent et une étape de polymérisation avec un taux d'agent polymérisant fixé à 5/4 équivalent (« protéines polymérisées F »).

### DESCRIPTION DETAILLEE DE L'INVENTION

Procédé de préparation d'un principe actif comprenant des protéines polymérisées.

Le procédé selon l'invention comprend les étapes consistant à solubiliser en phase aqueuse une farine, un isolat, concentrat, ou un tourteau riches en protéines à raison de 50 g/l à 500 g/l, de préférence à raison de 100 g/l à 300 g/l ;
à soumettre cette solution à une hydrolyse modérée et contrôlée à pH acide, à une température comprise entre 40°C et 80°C, en présence d'une enzyme, telle qu'une protéase de type endoprotéase, exoprotéase, exopeptidase, protéase-peptidase, aminopeptidase, carboxypeptidase ou de leurs mélanges ; le taux d'hydrolyse enzymatique est choisi de manière à maintenir un taux de protéines monomères de masse molaire de 20000 Da à 300000 Da.
à procéder ensuite à la séparation des phases soluble et insoluble et à inactiver l'enzyme ;
à polymériser les protéines récupérées par ajout d'un agent polymérisant multifonctionnel, de préférence comprenant au moins 10 g/l d'un initiateur de polymérisation choisi parmi les composés multifonctionnels tels que les polychlorures d'acides, les polyanhydrides d'acides, les polyisocyanates et les polythioisocyanates ; et
à séparer les protéines ainsi polymérisées par filtration, décantation, centrifugation et concentration.

### Principe d'une mesure d'effet tenseur :

Afin de caractériser l'efficacité cosmétique des protéines polymérisées produites par le procédé selon la présente invention sur les propriétés biomécaniques de la peau, on mesure principalement l'effet tenseur, à l'aide d'un dispositif vendu sous la dénomination commerciale « Cutomètre® » (Courage et Khazaka). Un tel dispositif comprend une sonde présentant un orifice de 2 mm. Cette sonde est appliquée sur la peau des avant bras des volontaires et une dépression constante de 350 mbars est maintenue dans cette sonde. On mesure la profondeur de pénétration de la peau dans cette sonde à travers l'orifice, sous l'effet de l'aspiration. La peau soumise à des dépressions se fatigue plus ou moins vite et les temps de réponse de même que les amplitudes obtenues permettent de déterminer notamment une déformation instantanée *Ue*, dite composante élastique, et une extensibilité *Uf*. Lorsque Ue et Uf diminuent, la peau est respectivement moins souple et moins extensible, donc plus tendue.

### Importance des différents paramètres et résultats.

### 1/ Intérêt de la polymérisation des protéines

L'étude suivante permet de comparer l'efficacité cosmétique de protéines non polymérisées et de protéines polymérisées telles qu'obtenues par le procédé selon l'invention.

Les protéines non polymérisées, notées « a » sont obtenues après solubilisation en phase aqueuse d'une matière riche en protéines comme par exemple, un isolat, un tourteau, une farine, un concentrat..., suivie d'une hydrolyse enzymatique contrôlée avec un taux d'enzymes fixé à 1 équivalent.

Les protéines polymérisées, notées «A» sont obtenues conformément à la présente invention en ajoutant une étape de fabrication avec 1 équivalent d'agent polymérisant aux protéines « a » obtenues précédemment.

Comparaison analytique des protéines non polymérisées et des protéines polymérisées (invention).

**Tableau 1**

| | Taux enzymatique | Agent polymérisant | Taux de matières sèches | Taux de protéines |
|---|---|---|---|---|
| Protéines a | 1 équivalent | 0 | 116 g/l | 110 g/l |
| Protéines polymérisées A (invention) | 1 équivalent | 1 équivalent | 115 g/l | 86 g/l |

Le taux de matières sèches est pratiquement identique pour les protéines notées a et A. Par contre, le taux de protéines est inférieur pour les protéines polymérisées A, puisqu'elles sont de taille plus importante (Tableau 1 et Figures 1 et 2).

### Comparaison de la taille des protéines notées a et A

Les tailles des protéines notées a et A sont déterminées par les profils chromatographiques et sont classées dans les 3 fractions F1, F2 et F3, déjà mentionnées ci-dessus.

**Tableau 2**

| | % de protéines dans les fractions | | |
|---|---|---|---|
| | F1 | F2 | F3 |
| Protéines a | 0% | 40% | 60% |
| Protéines polymérisées A (invention) | 45% | 30% | 25% |

Les protéines polymérisées notées A présentent une répartition de protéines de haut poids moléculaire complètement différente des protéines monomères notées a. (Tableau 2 et Figures 1 et 2).

### Comparaison de l'efficacité des protéines notées a (témoin) et A (invention) sur les propriétés biomécaniques de la peau.

L'influence de la polymérisation a été évaluée sur volontaires humains.

**Tableau 3**

| | -ΔUf | -ΔUe |
|---|---|---|
| Protéines a | 0,2% | 4,1% |
| | *p* = *0,304* | *p = 0,228* |
| Protéines | 3,4 % | 4,6 % |
| polymérisées A (invention) | *p = 0,056* | *p = 0,032* |

Les protéines polymérisées conformément à l'invention notées A présentent un effet tenseur significatif beaucoup plus important que celui des protéines non polymérisées notées a. L'étape additionnelle de polymérisation caractéristique du procédé selon l'invention apparaît donc déterminante pour l'effet technique recherché.

### 2/ Détermination du taux d'hydrolyse enzymatique des protéines monomères.

L'étude suivante permet de comparer l'influence sur le procédé de l'invention de l'application de différents taux enzymatiques tout en maintenant constant le taux d'agent polymérisant et de déterminer ainsi le taux d'hydrolyse enzymatique approprié permettant d'optimiser la taille des protéines monomères.

Comparaison analytique de 3 types de protéines polymérisées notées B, A et C avec différents taux d'hydrolyse enzymatique et un taux d'agent polymérisant fixé à 1 équivalent.

**Tableau 4**

| | Taux enzymatique | Agent polymérisant | Taux de matières sèches | Taux de protéines |
|---|---|---|---|---|
| Protéines Polymérisées B | 1/12 équivalent | 1 équivalent | 131 g/l | 124 g/l |
| Protéines polymérisées A (invention) | 1 équivalent | 1 équivalent | 115 g/l | 86 g/l |
| Protéines polymérisées C | 10/3 équivalent | 1 équivalent | 114 g/l | 86 g/l |

Les protéines polymérisées notées A et C présentent des taux de matières sèches et de protéines similaires. Par contre, les protéines polymérisées notées B possèdent un taux de protéines supérieur.

### Comparaison de la taille des protéines polymérisées notées B, A et C.

La taille des protéines notées B, A et C est déterminée par les profils chromatographiques et est classée dans les 3 fractions F1, F2 et F3.

**Tableau 5**

| | % de protéines dans les fractions | | |
|---|---|---|---|
| | F1 | F2 | F3 |
| Protéines B polymérisées | 22% | 61% | 17% |
| Protéines polymérisées A (invention) | 45% | 30% | 25% |
| Protéines polymérisées C | 34% | 32% | 34% |

Les protéines polymérisées notées A présentent le pourcentage le plus élevé de protéines de poids moléculaire supérieur à 500000 Da. (Tableau 5 et Figures 2, 4 et 6).

### Comparaison de l'efficacité des protéines polymérisées notées A, B et C sur les propriétés biomécaniques de la peau.

**Tableau 6**

| | -ΔUf | -ΔUe |
|---|---|---|
| Protéines polymérisées | 2,3% | 2,2% |
| B | *p = 0,090* | *p = 0,112* |
| Protéines polymérisées | 3,4 % | 4,6 % |
| A | *p = 0,056* | *p = 0,032* |
| (invention) | | |
| Protéines polymérisées | 2,3% | 1,0% |
| C | *p = 0,046* | *p = 0,169* |

Les protéines polymérisées notées B et C obtenues respectivement avec un taux enzymatique très faible, fixé à 1/12, et très important, fixé à 10/3, ne possèdent qu'une médiocre activité tenseur peu significative sur la peau.

A l'opposé les protéines polymérisées selon le procédé conforme à la présente invention, notées A, présentent un effet tenseur significatif. Il existe donc bien une relation optimale entre la taille des protéines monomères et leur activité tenseur.

Il apparaît déterminant de choisir un taux d'hydrolyse enzymatique de manière à maintenir une proportion d'environ 50/50 entre la fraction F2, de masse molaire entre 150000 Da et 500000 Da, et la fraction F3, de masse molaire inférieure à 150000 Da pour les protéines monomères.

Les pourcentages des fractions F1, F2 et F3 pour les protéines monomères notées a, b et c, avec les différents taux d'hydrolyse cités ci-dessus sont regroupés dans le tableau suivant :

| | F1 | F2 | F3 |
|---|---|---|---|
| Protéines b | 0% | 70% | 30% |
| Protéines a | 0% | 40% | 60% |
| Protéines c | 0% | 30% | 70% |

### 3/ Importance du taux d'agent polymérisant

L'étude suivante permet d'établir le taux d'agent polymérisant approprié pour optimiser l'activité cosmétique.

Les protéines polymérisées notées D sont obtenues avec un taux d'agent polymérisant faible et fixé à 5/8 d'équivalent.

A l'opposé, les protéines notées F sont obtenues avec un taux d'agent polymérisant élevé et fixé à 5/4 d'équivalent.

Comparaison analytique de 3 types de protéines polymérisées notées E, A et D avec différents taux d'agent polymérisant.

**Tableau 7**

| | Taux enzymatique | Agent polymérisant | Taux de matières sèches | Taux de protéines |
|---|---|---|---|---|
| Protéines Polymérisées D | 1 équivalent | 5/8 équivalent | 131 g/l | 124 g/l |
| Protéines polymérisées A (invention) | 1 équivalent | 1 équivalent | 115 g/l | 86 g/l |
| Protéines polymérisées E | 1 équivalent | 5/4 équivalent | 116 g/l | 67 g/l |

On observe que plus le taux d'agent polymérisant augmente, plus le taux de protéines diminue, pour des taux de matières sèches équivalents.

Comparaison de la taille des protéines polymérisées notées D, A et E.

La taille des protéines notées D, A et E est déterminée par les profils chromatographiques dans les fractions F1, F2 et F3.

**Tableau 8**

| | % de protéines dans les fractions | | |
|---|---|---|---|
| | F1 | F2 | F3 |
| Protéines D polymérisées | 22% | 45% | 33% |
| Protéines polymérisées A (invention) | 45% | 30% | 25% |
| Protéines polymérisées E | 84% | 6% | 10% |

L'augmentation du taux de protéines de la fraction F1 de très haut poids moléculaire (supérieur à 500000 Da) est proportionnelle à l'augmentation du taux d'agent polymérisant. (Tableau 8 et figures 2, 7 et 8).

### Comparaison de l'efficacité des protéines polymérisées notées A, D et E sur les propriétés biomécaniques de la peau.

**Tableau 9**

| | -ΔUf | -ΔUe |
|---|---|---|
| Protéines polymérisées D | 4,1% | 1,8% |
| | *p = 0,022* | *p* = *0,156* |
| Protéines polymérisées A | 3,4 % | 4,6 % |
| (invention) | *p = 0, 056* | *p* = *0,032* |
| Protéines polymérisées E | 1,9% | 2,3% |
| | *p* = *0, 219* | *p = 0,232* |

Les protéines polymérisées notées D, et E obtenues avec des faibles et forts taux d'agent polymérisant possèdent respectivement un faible effet peu significatif et aucun effet tenseur sur les propriétés de la peau.

A l'opposé, les protéines polymérisées conformément au procédé de l'invention , notées A, présentent un effet tenseur significatif.

Le taux d'agent polymérisant est de préférence choisi de manière à obtenir un pourcentage de fraction F1 supérieur à environ 30%.

Le pourcentage de 45% de protéines dans la fraction F1 de haut poids moléculaire (supérieur à 500000 Da) apparaît particulièrement avantageux.

### 4/ Importance du maintien d'une structure protéique du monomère.

L'étude suivante permet de déterminer la taille de la structure des protéines du monomère permettant d'optimiser l'efficacité cosmétique.

Les protéines polymérisées, notées F, sont obtenues avec un taux d'hydrolyse enzymatique important fixé à 10/3 équivalent ainsi qu'un taux d'agent polymérisant important fixé à 5/4 équivalent, dans le but de préparer des protéines polymérisées de tailles identiques à celles des protéines polymérisées A.

### Comparaison analytique de 2 types de protéines polymérisées notées F et A

**Tableau 10**

| | Taux enzymatique | Agent polymérisant | Taux de matières sèches | Taux de protéines |
|---|---|---|---|---|
| Protéines Polymérisées F | 10/3 équivalent | 5/4 équivalent | 119 g/l | 76 g/l |
| Protéines polymérisées A (invention) | 1 équivalent | 1 équivalent | 115 g/l | 86 g/l |

Les protéines polymérisées notées F présentent un taux de protéines légèrement inférieur à celui des protéines notées A.

### Comparaison de la taille des protéines polymérisées notées F et A.

La taille des protéines notées F et A est déterminée par les profils chromatographiques et est classée dans les 3 fractions F1, F2 et F3.

**Tableau 11**

| | % de protéines dans les fractions | | |
|---|---|---|---|
| | F1 | F2 | F3 |
| Protéines F | 42% | 40% | 18% |
| Protéines polymérisées A (invention) | 45% | 30% | 25% |

Les protéines polymérisées notées F et A présentent des pourcentages dans la fraction F1 pratiquement identiques.(Tableau 11 et Figures 2 et 9).

### Comparaison de l'efficacité des protéines notées F et A sur les propriétés biomécaniques de la peau.

**Tableau 12**

| | -ΔUf | -ΔUe |
|---|---|---|
| Protéines polymérisées F | 6,5% | 7,4% |
| | *p = 0,021* | *p* = *0,091* |
| Protéines polymérisées A | 3,4 % | 4,6 % |
| (invention) | *p = 0,056* | *p = 0,032* |

Les protéines polymérisées notées F et A bien que présentant des pourcentages de protéines dans la fraction F1 (d'un poids moléculaire supérieur à 500000 Da) pratiquement égaux, ne présentent pas la même efficacité. En effet seules les protéines polymérisées notées A présentent un effet tenseur significatif.

Cette étude montre qu'il existe une structure appropriée du monomère garantissant les effets sur les propriétés biomécaniques de la peau.

Dans ce qui suit sont donnés à titre purement illustratif et non limitatif des exemples de compositions cosmétiques conformes à la présente invention.

### Exemple 1 :

### Gel-Crème à effet tenseur

Arachidyl alcool/béhenyl alcool/arachidyl glucoside (= Montanov 202) : 3%
Isononyl isononanoate (= Lanol 99) : 2 %
Cétéaryl octanoate (=Lanol 1688) : 10%
Principe actif obtenu par le procédé selon l'invention : 3%
Polyacrylamide/ C₁₃₋₁₄ Isoparaffin/Laureth-7(= Sepigel 305) : 2%
Conservateur : 0,5 %
Eau : qsp 100%

Par application de ce gel-crème, la surface de la peau est tonique. Les rides semblent atténuées, la peau paraît liftée.

Exemple 2 :
Lait corporel soin du corps amincissant et lissant
Cyclométhicone (=DC 345) : 5%
Coco-Caprylate/Caprate (=Lanol 2681) : 2%
Isononyl isononanoate (=Lanol 99) : 3%
Principe actif obtenu par le procédé selon l'invention : 3%
C₁₃₋₁₄ Isoparaffin / Isostéaryl Isostéarate / Sodium polyacrylate / Polyacrylamide Polysorbate 60 (=Sepigel 501) : 3%
Actif amincissant : 3%
Glycérol : 2%
Ethanol : 8%
Conservateur : 0,5%
Eau : qsp 100%.

Par application de ce lait corporel la surface de la peau est lissée, les irrégularités semblent atténuées et la peau paraît plus lisse.

## Revendications

1. Procédé de préparation d'un principe actif à base de protéines polymérisées de haut poids moléculaire, ledit procédé comprenant les étapes consécutives suivantes consistant à
(i) solubiliser en phase aqueuse une matière riche en protéines, choisie parmi les farines, les isolats, les concentrats et les tourteaux riches en protéines naturelles végétales et/ou animales, et/ou marines ;
(ii) hydrolyser par voie enzymatique, avec une protéase de type endoprotéase, exoprotéase, exopeptidase, protéase-peptidase, aminopeptidase ou carbopeptidase ou un mélange de ces enzymes, de manière contrôlée et limitée la solution obtenue en (i), avec un taux d'hydrolyse enzymatique choisi de manière à maintenir des protéines monomères de masse molaire de 20000 Da à 300000 Da ; optionellement, inactiver le(s) enzyme(s) utilisée(s) ;
(iii) séparer les phases solubles et insolubles ; et
(iv) polymériser les protéines par ajout d'un agent polymérisant multifonctionnel choisi parmi les polychlorures d'acides, les polyanhydrides d'acides, les polyisocyanates et les polythioisocyanates.

2. Procédé selon la revendication 1, **caractérisé en ce que** le taux d'agent polymérisant est choisi de manière à obtenir une fraction des protéines polymérisées de masse molaire supérieure à 500000 Da.

3. Principe actif susceptible d'être obtenu par un procédé selon la revendication 1 ou 2, **caractérisé en ce que** les protéines polymérisées qu'il comprend présentent un taux de protéines supérieur à environ 30% dans la fraction de masse molaire supérieure à 500000 Da.

4. Principe actif selon la revendication précédente ou susceptible d'être obtenu par un procédé selon la revendication 1 ou 2, ayant un effet lissant, anti-rides et tenseur immédiat de la peau.

5. Composition cosmétique comprenant au moins un principe actif selon la revendication 3 ou 4, ou obtenu par le procédé selon l'une quelconque des revendications 1 ou 2.

6. Composition selon la revendication 5, **caractérisée en ce qu'**elle se présente sous forme d'une crème, d'un gel, d'un onguent, d'une lotion, d'un lait, d'une émulsion ou d'une solution.

## Claims

1. A method of preparing an active principle based on polymerised proteins with a high molecular weight, said method comprising the following consecutive steps consisting of:
(i) solubilising in aqueous phase a protein-rich material, chosen from flours, isolates, concentrates and cakes rich in natural vegetable and/or animal and/or marine proteins;
(ii) hydrolysing, by enzymatic method, with a protease of the endoprotease, exoprotease, exopeptidase, protease-peptidase, aminopeptidase or carbopeptidase type or a mixture of these enzymes, in a controlled and limited manner, the solution obtained at (i), with a degree of enzymatic hydrolysis chosen so as to maintain monomer proteins with a molar mass of 20,000 Da to 300,000 Da; optionally inactivating the enzyme(s) used;
(iii) separating the soluble and insoluble phases; and
(iv) polymerising the proteins by adding a multifunctional polymerising agent chosen from acid polychlorides, acid polyanhydrides, polyisocyanates and polythioisocyanates.

2. A method according to claim 1, **characterised in that** the level of polymerising agent is chosen to as to obtain a fraction of the polymerised proteins with a molar mass greater than 500,000 Da.

3. An active principle able to be obtained by a method according to claim 1 or 2, **characterised in that** the polymerised proteins that it comprises have a protein level greater than approximately 30% in the fraction with a molar mass greater than 500,000 Da.

4. An active principle according the preceding claim or able to be obtained by a method according to claim 1 or 2, having an immediate smoothing, anti-wrinkle and tightening effect on the skin.

5. A cosmetic composition comprising at least one active principle according to claim 3 or 4, or obtained by the method according to either one of claims 1 or 2.

6. A composition according to claim 5, **characterised in that** it is in the form of a cream, gel, ointment, lotion, milk, emulsion or solution.

## Patentansprüche

1. Verfahren zur Herstellung eines Wirkstoffs auf der Grundlage von polymerisierten Proteinen mit hohem Molekulargewicht, wobei das Verfahren die folgenden aufeinanderfolgenden Verfahrensschritte aufweist:
(i) Lösen in wässriger Lösung eines Materials, das reich an Proteinen ist und das unter den Mehlen, den Isolaten, den Konzentraten und den Presskuchen ausgewählt wird, die reich an natürlichen pflanzlichen und/oder tierischen und/oder maritimen Proteinen sind;
(ii) Hydrolysieren der in (i) erhaltenen Lösung auf kontrollierte und begrenzte Weise auf enzymatischem Weg mit einer Protease vom Typ Endoprotease, Exoprotease, Exopeptidase, Protease-Peptidase, Aminopeptidase oder Carbopeptidase oder einer Mischung dieser Enzyme mit einem Anteil an enzymatischer Hydrolyse, der derart gewählt ist, dass monomere Proteine mit einer Molekülmasse zwischen 20000 Da bis 300000 Da erhalten werden; optional Inaktivieren des (der) verwendeten Enzyms (Enzyme);
(iii)Trennen der löslichen und nichtlöslichen Phasen; und
(iv) Polymerisieren der Proteine durch Zugabe eines multifunktionellen polymerisierenden Mittels, das unter den Säurepolychloriden, den Säureanhydriden, den Polyisocyanaten und den Polythioisocyanaten ausgewählt wird.

2. Verfahren nach Anspruch 1, **dadurch gekennzeichnet, dass** der Anteil an polymerisierendem Mittel derart gewählt wird, dass ein Anteil von polymerisierten Proteinen mit einer Molekularmasse oberhalb von 500000 Da erhalten wird.

3. Wirkstoff, der durch ein Verfahren nach Anspruch 1 oder 2 erhältlich ist, **dadurch gekennzeichnet, dass** die darin enthaltenen polymerisierten Proteine mehr als 30% der Proteine in dem Anteil mit einer Molekularmasse oberhalb von 500000 Da ausmachen.

4. Wirkstoff nach dem vorhergehenden Anspruch, erhältlich durch ein Verfahren nach Anspruch 1 oder 2, der eine sofortige glättende, gegen Falten wirkende und straffende Wirkung auf die Haut hat.

5. Kosmetische Zusammensetzung, die weinigstens einen Wirkstoff nach Anspruch 3 oder 4 aufweist oder durch das Verfahren nach einem der Ansprüche 1 oder 2 erhalten worden ist.

6. Zusammensetzung nach Anspruch 5, **dadurch gekennzeichnet, dass** die Zusammensetzung eine Creme, ein Gel, eine Salbe, eine Lotion, eine Milch, eine Emulsion oder eine Lösung ist.
